# EUROPEAN PATENT SPECIFICATION

(11) **EP 2 257 190 B1**
(45) Date of publication and mention of the grant of the patent: **13.04.2016**
(21) Application number: 09713714.5
(22) Date of filing: 26.02.2009
(51) Int. Cl.: A23L 33/175, A61K 31/198, A23K 20/142

(54) **MIXED AQUEOUS SOLUTION OF L-LYSINE AND L-THREONINE**
WÄSSRIGE MISCHLÖSUNG VON L-LYSIN UND L-THREONIN
SOLUTION AQUEUSE MÉLANGÉE DE L-LYSINE ET L-THRÉONINE

(30) Priority: 29.02.2008 WO PCT/IB2008/001889
(43) Date of publication of application: 08.12.2010
(73) Proprietor: Ajinomoto Eurolysine S.a.s., 75817 Paris Cedex 17 (FR)
(72) Inventor: CROMBEZ, Mathilde, F-80084 Amiens Cedex 2 (FR); LEFEBVRE, François, F-80084 Amiens Cedex 2 (FR); CHIKAMORI, Takehiko, F-80084 Amiens Cedex 2 (FR); LE TUTOUR, Loïc, F-80084 Amiens Cedex 2 (FR); TORIDE, Yasuhiko, F-80084 Amiens Cedex 2 (FR); FUKE, Ichiro, 210-8681 (JP)
(74) Representative: Hart-Davis, Jason
(86) International application number: PCT/EP2009/052259
(87) International publication number: WO 2009/106558

(56) References cited:
- EP-A1- 1 068 804
- EP-B1- 0 022 361
- EP-B1- 0 534 865
- WO-A1-03/071878
- GB-A- 1 211 062
- US-A- 5 756 761
- US-A- 5 795 585
- US-A- 6 162 442

## Description

### Technical Field

The present invention relates to a mixed aqueous, solution of L-lysine and L-threonine essentially consisting of L-lysine, L-threonine and water and is used as a feed ingredient or the like.

### Background Art

A L-lysine based aqueous solution is already known as a feed ingredient containing an amino acid (see Patent document No. 1). It is also known that by adding an acid ion such as a sulfate ion into the L-lysine base aqueous solution to increase the solubility of L-lysine therein, a stable L-lysine base aqueous solution in which no crystals precipitate can be obtained (see Patent document No. 2). In addition, it is known that by electrodialyzing the L-lysine base aqueous solution to remove counter anions, a highly pure L-lysine base aqueous solution can be obtained (see Patent document No. 3).

Furthermore, it is preferred that a product form containing an amino acid for feed use be a liquid, due to the fact that the liquid form is more convenient in handling during its addition into the feed and that the uniform mixing can be more easily attained. In fact, liquid form amino acids are widely used in feed industry now. If the feed is distributed in a liquid form, it is generally preferred that the content of amino acid be high, which means the lower content of water because of the following reasons: 1) Lower transportation cost 2) Lower risk of microbial development during the storage of a feed after mixing 3) More suitable in formulating high nutrient density feed. For example, the L-lysine base aqueous solution is distributed at the concentration slightly below the saturation point to achieve the maximum concentration without the risk of the precipitation of crystals.

**Furthermore,** WO 03/071878 **discloses a feed supplement for increasing the plasma amino acid level of animals, including animal feed and liquid lysine base, where the liquid lysine base has a concentration of between about 45% and about 55%, and has a pH level of between about 9.5 and about 10.5.** EP 1 068 804 **discloses a method of preparing a lysine base dry powder, which can be used in an animal feed or premix.**

On the other hand, a liquid feed ingredient containing L-threonine is also desired, but has not been put into practical use yet.
[Patent document No. 1]
   EP 111628 B.
[Patent document No. 2]
   EP 1035109 B.
[Patent document No. 3]
   FR 2822396 B.

### Disclosure of Invention

Since L-threonine has low solubility unlike L-lysine, a solution containing only L-threonine tends to contain high amount of water, which results in high transportation costs and higher risk of microbial development. It is an object of the invention to provide a mixed aqueous solution of L-lysine and L-threonine with high total concentration of two amino acids, which can be prepared, sold, distributed, stored and used in stable conditions.

The present inventions comprises the following aspects:
(1) A mixed aqueous solution of L-lysine and L-threonine essentially consisting of L-lysine, L-threonine and water **as defined in appended claim 1, wherein said** solution has a viscosity of 3300 **mPa·s** (cp) or less **as measured** at 20°C and **at a pH of 11.3, wherein the mixed aqueous solution has** a pH of 10-13, **a concentration of L-threonine in the mixed aqueous solution is 40 g/100 a of water or more, a concentration of L-lysine in the mixed aqueous solution is 191 /100 a of water or less,** and a total concentration of L-lysine and L-threonine in the mixed aqueous solution **is** 70 g/100 g or more.
(2) The mixed aqueous solution of L-lysine and L-threonine of aspect (1), wherein the concentrations of L-lysine and L-threonine in the mixed aqueous solution are within the region delineated by the L-lysine line, the L-threonine line, the vertical axis and the horizontal axis in the mutual solubility diagram of L-lysine and L-threonine as measured at 20 °C provided that said region does not include said L-lysine line, L-threonine line, vertical axis and horizontal axis.
(3) The mixed aqueous solution of L-lysine and L-threonine of aspect (1), wherein the concentrations of L-lysine and L-threonine in the mixed aqueous solution are within the region delineated by the L-lysine line, the L-threonine line, the vertical axis and the horizontal axis in the mutual solubility diagram of L-lysine and L-threonine as measured at -5°C provided that said region does not include said L-lysine line, L-threonine line, vertical axis and horizontal axis.
(4) The mixed aqueous solution of L-lysine and L-threonine of any one of aspects (1)-(3), which has a viscosity of 2000 **mPa·s** (cp) or less **as measured** at 20 °C **and at a pH of 11.3.**
(5) The mixed aqueous solution of L-lysine and L-threonine of any one of aspects (1)-(4), which has a total concentration of L-lysine and L-threonine in the mixed aqueous solution of 100 g/100 g of water or more.
(6) The mixed aqueous solution of L-lysine and L-threonine of any one of aspects (1)-(5), which is prepared using solutions derived from a fermentation solution of L-lysine and/or L-threonine or a treated solution thereof.
(7) **An animal** feed ingredient wherein the mixed aqueous solution of L-lysine and L-threonine of any one of aspects (1)-(6) is formulated.
**(8) A method for preparing a mixed aqueous solution of L-lysine and L-threonine essentially consisting of L-lysine, L-threonine and water, said method being according to appended claim 8.**
**(9) A method for preparing a mixed aqueous solution of L-lysine and L-threonine essentially consisting of L-Iysine, L-threonine and water, said method being according to appended claim 9.**
**(10) Use of the mixed aqueous solution of L-lysine and L-threonine according to any one of aspects (1)-(6) in the preparation of animal feed.**

According to the present invention, a mixed aqueous solution of L-lysine and L-threonine can be put into practical use, which is stable and concentrated, easy to handle with low viscosity and thus can be applied to a compound feed.

### Brief Description of Drawings

Figure 1 shows a mutual solubility diagram of L-lysine and L-threonine at 20°C and pH 11.3.
Figure 2 shows a mutual solubility diagram of L-lysine and L-threonine at -5°C and pH 12.
Figure 3 shows temperature dependences of a mutual solubility of L-lysine and L-threonine.

### Best Mode for Carrying Out the Invention

L-lysine and L-threonine used in the present inventions as raw materials may be an aqueous solution containing each amino acid or each amino acid crystal, or alternatively a mixed solution of L-lysine and L-threonine or mixed crystal of L-lysine and L-threonine may be used. Generally, the origin of the above amino acid is not restricted to a specific one, but from the viewpoint of physiological safety or the like, it is preferred that the above amino acid be a raw material prepared using a fermentation method or an enzymatic method, said material is purified before use. The purity of the L-lysine raw material is preferably 95 % or more in dry matter, while the purity of the L-threonine raw material is preferably 98.5 % or more in dry matter. In addition, these raw materials may contain minerals such as potassium, magnesium, calcium etc., but the total amount of minerals is preferably 2400 ppm or less. This is so that the risk of precipitation of minerals can be minimized. The mixed solution of L-lysine and L-threonine according to the present invention is generally used at the temperature range of -5 to 60°C. Those skilled in the art may determine said temperature for the product of the present invention considering a manufacturing temperature, temperature of the area where the product is sold and distributed, storage temperature and operating temperature to keep the mixed solution in the stable state in which insoluble substances such as crystals are not precipitated. If necessary, a storage tank with an insulating jacket may be used to avoid the precipitation. Generally, the mixed solution is distributed and used at a temperature between -5°C and ambient, around 20°C, and it is not preferred to use it at -5°C or less from the viewpoint of the precipitation of crystal and it is not preferred to use it at 60°C or more from the viewpoint of the generation of decomposed material by amino-carbonyl reaction, said reaction occurs especially if raw materials prepared using a fermentation method or an enzymatic method are used. In connection to this, it has been confirmed that if the mixed solution saturated with L-lysine and L-threonine at -5°C is heated to a higher temperature, insoluble substances such as crystals are not precipitated.

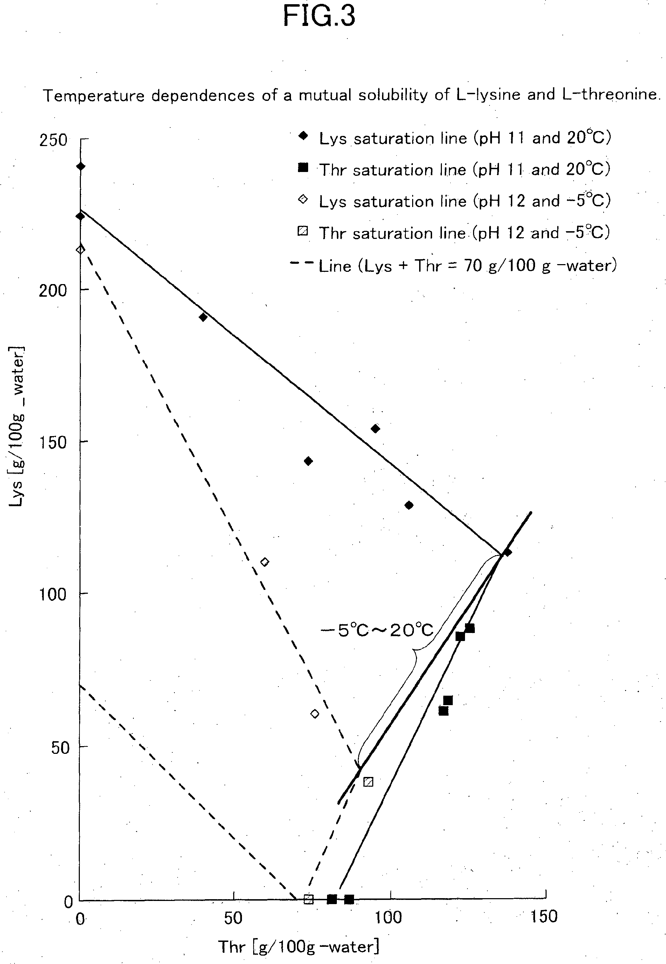

As mentioned above, although the mixed solution of L-lysine and L-threonine according to the present invention is generally used at the range of -5 to 60°C, the mutual solubility value of the mixed solution saturated with both L-lysine and L-threonine increases with an increase of temperature. In connection to this, the mutual solubility values at the temperature between -5°C and 20°C can be estimated from those at -5°C and 20°C as shown in Fig. 3. Since pH 11.3 (20°C) and pH 12 (-5°C) are very near each other, these pH values are considered the same values when preparing Fig. 3.

The range of pH of the mixed solution of L-lysine and L-threonine according to the present invention is restricted to from 10 to 13. A pH of less than 10 is not preferred because of the low solubility of L-threonine and a pH of more than 13 is not preferred because of the handling difficulty when using the mixed solution as a feed ingredient (i.e. it is designated as a hazardous material). Although a pH variation between 10 and 13 is accompanied by a mutual solubility variation and a viscosity variation, a mutual solubility diagram is easily prepared according to the method described in the present specification.

In order to control the pH value, if alkali is added to the solution, alkali metal or alkaline earth metal such as caustic soda, caustic potash or the like is used, and if acid is added to the solution, sulfuric acid, acetic acid or the like is used.

The total concentration of L-lysine and L-threonine in the mixed aqueous solution of 70 g/100 g of water or more, results in a feed solution containing a high level of L-lysine and L-threonine.

As described below, the present inventors have newly found that in the mixed solution of L-lysine and L-threonine according to the present invention, the viscosity thereof changes significantly depending on whether or not the L-lysine concentration is higher than the L-lysine line. In said mixed solution, from the viewpoint of workability and the like it is **chosen in the present invention** that the viscosity thereof **as measured** at 20°C **and at a pH of 11.3** be 3300 **mPa·s** (cp) or less, preferably 3000 cp or less. In addition, from the viewpoint of distribution, storage and the like it is preferred that the viscosity thereof at -5°C be 3300 cp or less, more preferably 3000 cp or less. That is, by keeping the viscosity of the mixed solution 3000 cp or less, stability of the mixed solution by distribution and storage can more easily be increased. Therefore, it is understood that 3300 cp or less is preferred, 3000 cp or less is more preferred.

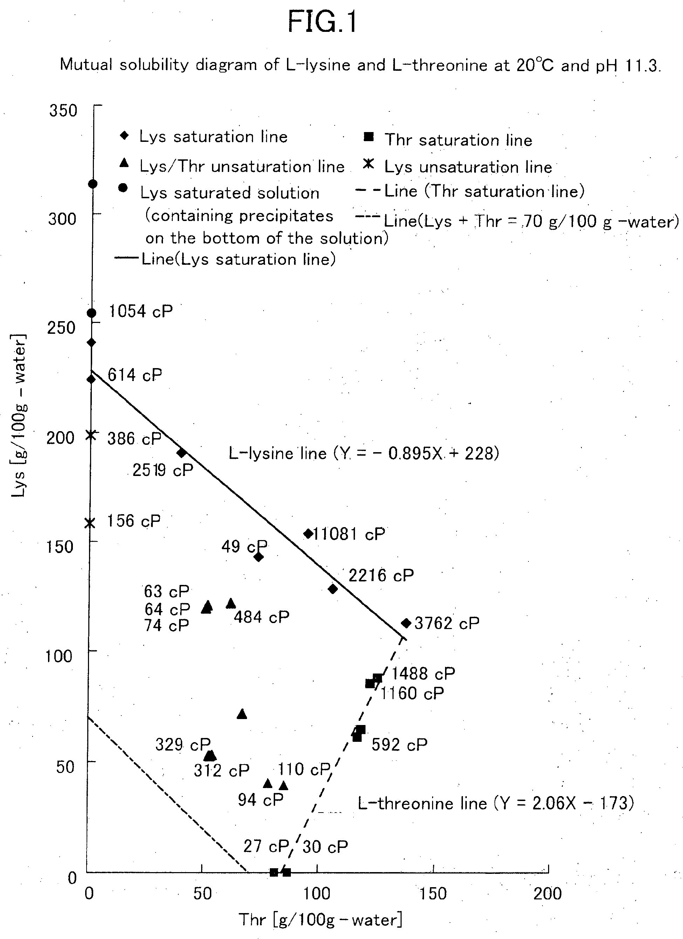

As an example of a mutual solubility diagram at room temperature, for example as shown in Fig. 1 regarding Example 1, a L-lysine line can be specified as Y=0.895X+228 and a L-threonine line can be specified as Y=2.06X-173 at 20°C and pH 11.3.

As an example of a mixed solution within the scope of the present invention, a mixed solution containing 191 g/100 g of water of L-lysine and 40 g/100 g of water of L-threonine, which are slightly lesser concentrations than the L-lysine line, do not precipitate any crystals, has a viscosity of 2519 cp and is a stable solution having an excellent handleability.

On the other hand, as an example of a mixed solution outside of the scope of the present invention, a mixed solution containing 154 g/100 g of water of L-lysine and 95 g/100 g of water of L-threonine, which are higher concentrations than the L-lysine line, precipitates crystals containing L-lysine which set in a gel form, has a viscosity reaching up to 11081 cp and is a completely uncontrollable liquid.

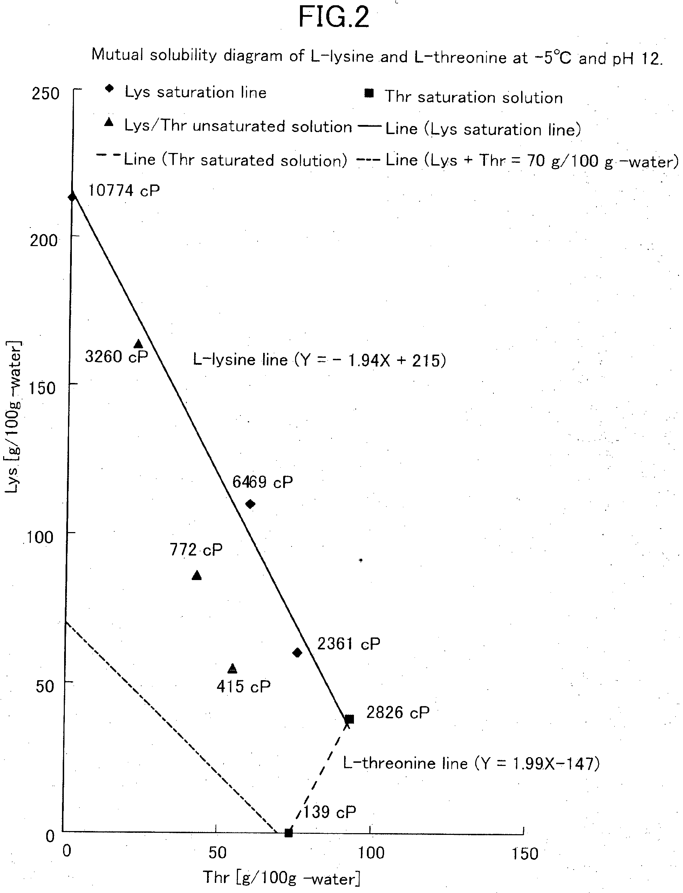

As an example of a mutual solubility diagram at a lower temperature, for example as shown in Fig. 2 regarding Example 2, a L-lysine line can be specified as Y=-1.94X+215 and a L-threonine line can be specified as Y=1.99X-147 at -5°C and pH 12.

As an example of a mixed solution within the scope of the present invention, a mixed solution containing 60 g/100 g of water of L-lysine and 76 g/100 g of water of L-threonine, which are slightly lesser concentrations than the L-lysine line, do not precipitate any crystals, has a viscosity of 2361 cp and is a stable solution having an excellent handleability at -5°C.

On the other hand, as an example of a mixed solution outside of the scope of the present invention, a mixed solution containing 110 g/100 g of water of L-lysine and 60 g/100 g of water of L-threonine, which are higher concentrations than the L-lysine line, precipitates crystals containing L-lysine which set in a gel form, has a viscosity reaching up to 6469 cp and is a completely uncontrollable liquid.

In the right region of the L-threonine line in which the solution contains a large amount of L-threonine, crystals containing L-threonine precipitate on the bottom of the solution.

Furthermore, in the present invention, from the viewpoint of handleability such as enabling the solution to spray when the solution is mixed with a feed, it is preferred that the viscosity of the mixed solution be 2000 cp or less at a specific temperature between -5°C and 60°C, for example 60°C, in particular 20°C.

In addition, from the viewpoint of reducing the content of water, that is: 1) Lower transportation cost 2) Lower risk of microbial development during the storage of a feed after mixing 3) More suitable in formulating high nutrient density feed, it is preferred that the total concentration of L-lysine and L-threonine be 100 g/100 g of water or more in the mixed aqueous solution.

For example, the above mutual solubility diagram used in the present inventions can be determined as follows.

By using a method in which L-threonine is added into a saturated solution of L-lysine at a predetermined pH and temperature or L- lysine is added into a saturated solution of L-threonine at a predetermined pH and temperature, or a method comprising concentrating or cooling a mixed aqueous solution of L-lysine and L-threonine having a variety of mix ratios prepared at a predetermined pH and temperature to precipitate crystals, the resulting crystals are separated from the mother liquor and then a solubility diagram is plotted setting the concentration of L-lysine in the mother liquor on the horizontal axis and the concentration of L-threonine in the mother liquor on the vertical axis. A line obtained using linear approximation of saturation points at which gelatinous L-lysine is precipitated is defined as a "L-lysine line" and a line obtained using linear approximation of saturation points at which needle-shaped L-threonine is precipitated is defined as a "L-threonine line" in the mutual solubility diagram. Data of the mutual solubility at pH 11.3 and 20°C, and at pH 12 and -5°C are shown in the following tables.

**Table 1: the mutual solubility of L-lysine and L-threonine at pH 11.3 and 20°C**

| No. | Lys [g/100 g of water] | Thr [g/100 g of water] | Remarks |
|---|---|---|---|
| 1 | 241 | 0 | Lys line |
| 2 | 224 | 0 | |
| 3 | 191 | 40 | |
| 4 | 143 | 73 | |
| 5 | 154 | 95 | |
| 6 | 129 | 106 | |
| 7 | 113 | 137 | |
| 8 | 0 | 81 | Thr line |
| 9 | 0 | 87 | |
| 10 | 61 | 117 | |
| 11 | 64 | 118 | |
| 12 | 85 | 122 | |
| 13 | 88 | 125 | |

**Table 2: the mutual solubility of L-lysine and L-threonine at pH 12 and -5°C**

| No. | Lys [g/100 g of water] | Thr [g/100 g of water] | Remarks |
|---|---|---|---|
| . 1 | 213 | 0 | Lys line |
| 2 | 110 | 60 | |
| 3 | 60 | 76 | |
| 4 | 0 | 74 | Thr line |
| 5 | 38 | 93 | |

In accordance with the above mutual solubility diagram, **in the present invention** it is **chosen** from the viewpoint of handleability that the total concentration of L-lysine and L-threonine be 70 g/100 g of water or more, and more preferably 100 g/100 g of water or more.

The viscosity was measured using a rotational viscometer (Rheomat RM 180, Metller Toledo) and the measure system DIN 53019 regarding saturated solutions of L-lysine containing L-threonine in the predetermined concentrations, saturated solutions of L-threonine containing L-lysine in the predetermined concentrations and mixed solutions of L-lysine and L-threonine at the predetermined concentrations. Data of the mixed solutions at pH 11.3 and 20°C, and pH 12 and -5°C are shown in the following tables.

**Table 3: the viscosity of the mixed solutions of L-lysine and L-threonine at pH 11.3 and 20°C**

| No. | Lys [g/100 g of water] | Thr [g/100 g of water] | Viscosity [cp] |
|---|---|---|---|
| 1 | 224 | 0 | 614 |
| 2 | 191 | 40 | 2519 |
| 3 | 143 | 73 | 49 |
| 4 | 154 | 95 | 11081 |
| 5 | 129 | 106 | 2216 |
| 6 | 113 | 137 | 3762 |
| 7 | 0 | 81 | 27 |
| 8 | 0 | 87 | 30 |
| 9 | 61 | 117 | 592 |
| 10 | 85 | 122 | 1160 |
| 11 | 88 | 125 | 1488 |
| 13 | 53 | 54 | 63 |
| 14 | 53 | 53 | 64 |
| 15 | 53 | 53 | 74 |
| 17 | 40 | 78 | 94 |
| 18 | 39 | 85 | 110 |
| 19 | 121 | 52 | 312 |
| 20 | 120 | 51 | 329 |
| 21 | 122 | 61 | 484 |
| 22 | 158 | 0 | 156 |
| 23 | 199 | 0 | 386 |
| 24 | 254 | 0 | 1054 |

**Table 4: the viscosity of the mixed solutions of L-lysine and L-threonine at pH 12 and -5°C**

| No. | Lys [g/100 g of water] | Thr [g/100 g of water] | Viscosity [cp] |
|---|---|---|---|
| 1 | 213 | 0 | 10774 |
| 2 | 110 | 60 | 6469 |
| 3 | 60 | 76 | 2361 |
| 4 | 0 | 74 | 139 |
| 5 | 38 | 93 | 2826 |
| 6 | 55 | 55 | 415 |
| 7 | 86 | 43 | 772 |
| 8 | 164 | 22 | 3260 |

Thus, since the mutual solubility can be identified, the mixed aqueous solution of L-lysine and L-threonine having the viscosity of 3300 cp or less can be obtained if the pH is determined to be 10 to 13, the total concentration of L-lysine and L-threonine is determined to be 70 g/100 g of water or more and the concentration of L-threonine is determined to be the specific concentration within the area of the mutual solubility.

The mixed aqueous solution of L-lysine and L-threonine according to the present invention can be used as an animal feed ingredient.

A solution prepared using an appropriate mix ratio of L-lysine and L-threonine (for example, 25 wt.% of L-threonine and 25 wt.% of L-lysine) is usually added in the amount of about 1 to 5 kg into one ton of animal feed using a spray nozzle. Because two kinds of required amino acids are accurately and easily added into animal feed with a single mixing, it is expected to be more useful as an animal feed ingredient than an aqueous solution or granulated dry crystals, containing L-lysine or L-threonine alone.

The present invention will be explained in more detail with reference to the following specific Examples in which the analysis of L-lysine and L-threonine was made by AOAC official method 999.13 (AOAC Official Methods of Analysis (2005) - Animal feed, Chapter 4, p20-24).

### Examples

### Example 1: Example at 20°C and pH 11.3

A 763.69 g quantity of 50% L-lysine solution and a 50.12 g quantity of L-threonine crystal, which were obtained from a commercial source (Ajinomoto Eurolysine S.A.S., (50% L-lysine; Lot 6256),(L-threonine; Lot 6255)), were mixed in a 1 l glass beaker. A 61.83 g quantity of 50% caustic soda (solid sodium hydroxide; Merck KGaA, reagent grade (purity >99%)) was then added to adjust the pH to 11 at room temperature. This solution was concentrated to about 1.6 fold by using a rotary evaporator (pressure: 100mbar, water bath temperature: 60°C). As a result, precipitation of crystals was observed. The slurry was stirred overnight at room temperature (20°C) so that the saturated solution does not become super saturated. Then, the saturated solution and crystals were separated at 20°C by centrifugation at 4000 rpm for 30 min (J2-21 M/E-Beckman, rotor JA-14). The viscosity and the contents of L-lysine, L-threonine, sodium, and water in the saturated solution were measured by analysis under the following conditions:
L-lysine content: Amino Acid Analyzer AMINOTAC JLC-500/V (JEOL)
L-threonine content: Amino Acid Analyzer AMINOTAC JLC-500/V (JEOL)
Viscosity: a rotational viscometer (Rheomat RM 180, Metller Toledo) and the measure system DIN 53019
Sodium content: Ion Chromatography DX320 (DIONEX)
Water content: Drying in the oven at 105°C overnight
Then, the L-lysine and L-threonine content in the saturated solution were plotted on the graph. There are three regions with crystals precipitated in the saturated solution, the region containing L-lysine alone, the region containing both L-lysine and L-threonine, the region containing L-threonine alone. And if separated crystals corresponding to the saturated solution were determined as L-lysine, these plots were defined as "L-lysine line" on a straight line. And if separated crystals corresponding to the saturated solution were determined as L-threonine, these plots were defined as "L-threonine line" on a straight line.

### Example 2: Example at -5°C and pH 12

A 514.20 g quantity of L-lysine solution, which had a L-lysine concentration of 61.46% and a pH which was adjusted to 10.98 at 20°C, and a 441.79g quantity of a L-threonine solution, which had L-threonine concentration of 42.93% and a pH which was adjusted to 11.02 at 20°C, were mixed in a 1 l glass beaker. The 61.46% L-lysine solution was obtained from a commercial source (Ajinomoto Eurolysine S.A.S., (50% L-lysine; Lot 6256)) after evaporation using a rotary evaporator (pressure: 100mbar, water bath temperature: 60°C), and the 42.93% L-threonine solution was also obtained from a commercial one (Ajinomoto Eurolysine S.A.S., (L-threonine; Lot 7158). The resulting solution mixed from L-lysine and L-threonine was stirred at room temperature overnight and cooled down to -5°C. The solution was maintained at -5°C under agitation for 48 hours, and then under static conditions overnight. As a result, precipitation of crystals was observed. Then, the saturated solution and crystals were separated at -5°C by centrifugation at 4000 rpm for 30 min (J2-21 M/E-Beckman, rotor JA-14). The viscosity and the contents of L-lysine, L-threonine, sodium, and water in saturated solution were measured by analysis under the following conditions:
L-lysine content: Amino Acid Analyzer AMINOTAC JLC-500/V (JEOL)
L-threonine content: Amino Acid Analyzer AMINOTAC JLC-500/V (JEOL)
Viscosity: a rotational viscometer (Rheomat RM 180, Metller Toledo) and the measure system DIN 53019
Sodium content: Ion Chromatography DX320 (DIONEX)
Water content: Drying in the oven at 105°C overnight
Then, the L-lysine and L-threonine contents in the saturated solution were plotted on the graph. There are three regions with crystals precipitated in the saturated solution, the region containing L-lysine alone, the region containing both L-lysine and L-threonine, the region containing L-threonine alone. If separated crystals corresponding to the saturated solution were determined as L-lysine by gel, these plots were defined as "L-lysine line" on a straight line. And if separated crystals corresponding to the saturated solution were determined as L-threonine by needle-like crystals, these plots were defined as "L-threonine line" on a straight line.

### Industrial Applicability

The present invention is a mixed aqueous solution of L-lysine and L-threonine which is stable and concentrated and which has good handleability and thus can be used as an animal feed ingredient.

## Claims

1. A mixed aqueous solution of L-lysine and L-threonine essentially consisting of L-lysine, L-threonine and water, wherein said solution has a viscosity of 3300 mPa·s (3300 cp) or less as measured at 20°C and at a pH of 11.3, wherein the mixed aqueous solution has a pH of 10-13, a concentration of L-threonine in the mixed aqueous solution is 40 g/100 g of water or more, a concentration of L-lysine in the mixed aqueous solution is 191 g/100 g of water or less, and a total concentration of L-lysine and L-threonine in the mixed aqueous solution is 70 g/100 g of water or more.

2. The mixed aqueous solution of L-lysine and L-threonine according to claim 1, wherein the concentrations of L-lysine and L-threonine in the mixed aqueous solution are within the region delineated by the L-lysine line, the L-threonine line, the vertical axis and the horizontal axis in the mutual solubility diagram of L-lysine and L-threonine as measured at 20°C provided that said region does not include said L-lysine line, L-threonine line, vertical axis and horizontal axis.

3. The mixed aqueous solution of L-lysine and L-threonine according to claim 1, wherein the concentrations of L-lysine and L-threonine in the mixed aqueous solution are within the region delineated by the L-lysine line, the L-threonine line, the vertical axis and the horizontal axis in the mutual solubility diagram of L-lysine and L-threonine as measured at -5°C provided that said region does not include said L-lysine line, L-threonine line, vertical axis and horizontal axis.

4. The mixed aqueous solution of L-lysine and L-threonine according to any one of claims 1-3, which has a viscosity of 2000 mPa·s (2000 cp) or less as measured at 20°C and at a pH of 11.3.

5. The mixed aqueous solution of L-lysine and L-threonine according to any one of claims 1-4, which has a total concentration of L-lysine and L-threonine in the mixed aqueous solution of 100 g/100 g of water or more.

6. The mixed aqueous solution of L-lysine and L-threonine according to any one of claims 1-5, which is prepared using solutions derived from a fermentation solution of L-lysine and/or L-threonine or a treated solution thereof.

7. An animal feed ingredient consisting of the mixed aqueous solution of L-lysine and L-threonine according to any one of claims 1-6.

8. A method for preparing a mixed aqueous solution of L-lysine and L-threonine essentially consisting of L-lysine, L-threonine and water, said method comprising the steps of:
(i) a step to create an expression of a mutual solubility at a specified pH and temperature; and
(ii) on the basis of the information of the said step (i), a step to prepare a mixed aqueous solution of L-lysine, L-threonine and water such that the mixed aqueous solution has a viscosity of 3300 mPa·s (3300 cp) or less as measured at 20°C and at a pH of 11.3, wherein the mixed aqueous solution has a pH of 10-13, a concentration of L-threonine in the mixed aqueous solution is 40 g/100 g of water or more, a concentration of L-lysine in the mixed aqueous solution is 191 g/100 g of water or less, and a total concentration of L-lysine and L-threonine in the mixed aqueous solution is 70 g/100 g of water or more.

9. The method according to claim 8, wherein the step (i) comprises a step to create an expression of a mutual solubility at 20°C and a pH of 11.3, wherein a L-lysine line can be specified as Y=-0.895X+228 and a L-threonine line can be specified as Y=2.06X-173, wherein Y represents the concentration of L-lysine and X represents the concentration of L-threonine.

10. Use of the mixed aqueous solution of L-lysine and L-threonine according to any one of claims 1-6 in the preparation of animal feed.

## Patentansprüche

1. Gemischte wässrige Lösung von L-Lysin und L-Threonin, die im Wesentlichen aus L-Lysin und L-Threonin und Wasser besteht, wobei die Lösung eine bei 20°C und einem pH-Wert von 11,3 gemessene Viskosität von 3300 mPa·s (3300 cp) oder weniger aufweist, wobei die gemischte wässrige Lösung einen pH-Wert von 10 bis 13 aufweist, eine Konzentration von L-Threonin in der gemischten wässrigen Lösung 40 g/100 g Wasser oder mehr ist, eine Konzentration von L-Lysin in der gemischten wässrigen Lösung 191 g/100 g Wasser oder weniger ist und eine Gesamtkonzentration von L-Lysin und L-Threonin in der gemischten wässrigen Lösung 70 g/100 g Wasser oder mehr ist.

2. Gemischte wässrige Lösung aus L-Lysin und L-Threonin nach Anspruch 1, wobei die Konzentrationen von L-Lysin und L-Threonin in der gemischten wässrigen Lösung innerhalb des Bereichs liegen, der durch die L-Lysinlinie, die L-Threonin-linie, die vertikale Achse und die horizontale Achse in dem Diagramm der bei 20°C gemessenen gegenseitigen Löslichkeit von L-Lysin und L-Threonin beschrieben wird, mit der Maßgabe, dass dieser Bereich die L-Lysinlinie, die L-Threoninlinie, die vertikale Achse und die horizontale Achse nicht umfasst.

3. Gemischte wässrige Lösung von L-Lysin und L-Threonin nach Anspruch 1, wobei die Konzentrationen von L-Lysin und L-Threonin in der gemischten wässrigen Lösung innerhalb des Bereichs liegen, der durch die L-Lysinlinie, die L-Threonin-linie, die vertikale Achse und die horizontale Achse in dem Diagramm der bei -5°C gemessenen gegenseitigen Löslichkeit von L-Lysin und L-Threonin beschrieben wird, mit der Maßgabe, dass der Bereich die L-Lysinlinie, L-Threoninlinie, die vertikale Achse und die horizontal Achse nicht umfasst.

4. Gemischte wässrige Lösung von L-Lysin und L-Threonin nach einem der Ansprüche 1 bis 3, die eine bei 20°C und einem pH-Wert von 11,3 gemessene Viskosität von 2000 mPa·s (2000 cp) oder weniger aufweist.

5. Gemischte wässrige Lösung von L-Lysin und L-Threonin nach einem der Ansprüche 1 bis 4 , die eine Gesamtkonzentration von L-Lysin und L-Threonin in der gemischten wässrigen Lösung von 100 g/100 g Wasser oder mehr aufweist.

6. Gemischte wässrige Lösung von L-Lysin und L-Threonin nach einem der Ansprüche 1 bis 5, hergestellt unter Verwendung von Lösungen, die von einer Fermentationslösung von L-Lysin und/oder L-Threonin abgeleitet sind, oder einer behandelten Lösung davon.

7. Tierfutterbestandteil, der aus der gemischten wässrigen Lösung von L-Lysin und L-Threonin nach einem der Ansprüche 1 bis 6 besteht.

8. Verfahren zum Herstellen einer gemischten wässrigen Lösung von L-Lysin und L-Threonin, die im Wesentlichen aus L-Lysin, L-Threonin und Wasser besteht, wobei das Verfahren die folgenden Stufen umfasst:
(i) eine Stufe, in der die gegenseitige Löslichkeit bei einem bestimmten pH-Wert und einer bestimmten Temperatur ermittelt wird; und
(ii) auf der Grundlage der Information der Stufe (i) eine Stufe, in der eine gemischte wässrige Lösung von L-Lysin, L-Threonin und Wasser so hergestellt wird, dass die gemischte wässrige Lösung eine bei 20°C und einem pH-Wert von 11,3 gemessene Viskosität von 3300 mPa·s (3300 cp) oder weniger aufweist, wobei die gemischte wässrige Lösung einen pH-Wert von 10 bis 13 aufweist, die Konzentration von L-Threonin in der gemischten wässrigen Lösung 40 g/100 g Wasser oder mehr ist, die Konzentration von L-Lysin in der gemischten wässrigen Lösung 191 g/100 g Wasser oder weniger ist und die Gesamtkonzentration von L-Lysin und L-Threonin in der gemischten wässrigen Lösung 70 g/100 g Wasser oder mehr ist.

9. Verfahren nach Anspruch 8, wobei die Stufe (i) eine Stufe umfasst, in der die gegenseitige Löslichkeit bei 20°C und einem pH-Wert von 11,3 ermittelt wird, wobei eine L-Lysinlinie als Y = -0,895X + 228 und eine L-Threoninlinie als Y = 2,06X - 173 angegeben werden kann, wobei Y die Konzentration von L-Lysin und X die Konzentration von L-Threonin darstellt.

10. Verwendung der gemischten wässrigen Lösung von L-Lysin und L-Threonin nach einem der Ansprüche 1 bis 6, bei der Herstellung eines Tierfuttermittels.

## Revendications

1. Solution aqueuse mixte de L-lysine et de L-thréonine consistant essentiellement en L-lysine, en L-thréonine et en eau, dans laquelle ladite solution présente une viscosité de 3 300 mPa.s (3 300 cP) ou moins mesurée à 20 °C et à un pH de 11,3, dans laquelle la solution aqueuse mixte présente un pH de 10 à 13, une concentration en L-thréonine dans la solution aqueuse mixte de 40 g/100 g d'eau ou plus, une concentration de L-lysine dans la solution aqueuse mixte de 191 g/100 g d'eau ou moins, et une concentration totale de L-lysine et de L-thréonine dans la solution aqueuse mixte de 70 g/100 g d'eau ou plus.

2. Solution aqueuse mixte de L-lysine et de L-thréonine selon la revendication 1, dans laquelle les concentrations de L-lysine et de L-thréonine dans la solution aqueuse mixte sont à l'intérieur de la région délimitée par la ligne de la L-lysine, la ligne de la L-thréonine, l'axe vertical et l'axe horizontal du diagramme de solubilité mutuelle de la L-lysine et de la L-thréonine, mesurée à 20 °C, à la condition que ladite région n'inclut pas ladite ligne de la L-lysine, ladite ligne de la L-thréonine, ledit axe vertical et ledit axe horizontal.

3. Solution aqueuse mixte de L-lysine et de L-thréonine selon la revendication 1, dans laquelle les concentrations de L-lysine et de L-thréonine dans la solution aqueuse mixte sont à l'intérieur de la région délimitée par la ligne de la L-lysine, la ligne de la L-thréonine, l'axe vertical et l'axe horizontal du diagramme de solubilité mutuelle de la L-lysine et de la L-thréonine, mesurée à -5 °C, à la condition que ladite région n'inclut pas ladite ligne de la L-lysine, ladite ligne de la L-thréonine, ledit axe vertical et ledit axe horizontal.

4. Solution aqueuse mixte de L-lysine et de L-thréonine selon l'une quelconque des revendications 1 à 3, qui présente une viscosité de 2 000 mPa.s (2 000 cP) ou moins mesurée à 20 °C et à un pH de 11,3.

5. Solution aqueuse mixte de L-lysine et de L-thréonine selon l'une quelconque des revendications 1 à 4, qui présente une concentration totale de L-lysine et de L-thréonine dans la solution aqueuse mixte de 100 g/100 g d'eau ou plus.

6. Solution aqueuse mixte de L-lysine et de L-thréonine selon l'une quelconque des revendications 1 à 5, qui est préparée en utilisant des solutions dérivées d'une solution de fermentation de L-lysine et/ou L-thréonine ou d'une solution traitée de celles-ci.

7. Ingrédient d'aliment pour animaux consistant en la solution aqueuse mixte de L-lysine et de L-thréonine selon l'une quelconque des revendications 1 à 6.

8. Procédé de préparation d'une solution aqueuse mixte de L-lysine et de L-thréonine consistant essentiellement en L-lysine, en L-thréonine et en eau, ledit procédé comprenant les étapes suivantes :
(i) une étape destinée à créer une expression d'une solubilité mutuelle à un pH et une température spécifiés ; et
(ii) sur la base des informations de ladite étape (i), une étape destinée à préparer une solution aqueuse mixte de L-lysine, de L-thréonine et d'eau, de telle sorte que la solution aqueuse mixte présente une viscosité de 3 300 mPa.s (3 300 cP) ou moins mesurée à 20 °C et à un pH de 11,3, dans laquelle la solution aqueuse mixte présente un pH de 10 à 13, une concentration de L-thréonine dans la solution aqueuse mixte de 40 g/100 g d'eau ou plus, une concentration de L-lysine dans la solution aqueuse mixte de 191 g/100 g d'eau ou moins, et une concentration totale de L-lysine et de L-thréonine dans la solution aqueuse mixte de 70 g/100 g d'eau ou plus.

9. Procédé selon la revendication 8, dans lequel l'étape (i) comprend une étape pour créer une expression d'une solubilité mutuelle à 20 °C et à un pH de 11,3, dans laquelle une ligne de L-lysine peut être spécifiée comme Y = -0,895 X + 228 et une ligne de L-thréonine peut être spécifiée comme Y = 2,06 X - 173, où Y représente la concentration de L-lysine et X représente la concentration de L-thréonine.

10. Utilisation de la solution aqueuse mixte de L-lysine et de L-thréonine selon l'une quelconque des revendications 1 à 6 dans la préparation d'un aliment pour animaux.
